(19) **Europäisches Patentamt / European Patent Office / Office européen des brevets**

(11) **EP 1 989 268 B1**

(12) **EUROPÄISCHE PATENTSCHRIFT**

(45) Veröffentlichungstag und Bekanntmachung des Hinweises auf die Patenterteilung:
**20.01.2010 Patentblatt 2010/03**

(21) Anmeldenummer: **07704546.6**

(22) Anmeldetag: **13.02.2007**

(51) Int Cl.:
***C09D 151/00*** ^(2006.01)

(86) Internationale Anmeldenummer:
**PCT/EP2007/051372**

(87) Internationale Veröffentlichungsnummer:
**WO 2007/096272 (30.08.2007 Gazette 2007/35)**

(54) **VERWENDUNG VON FARBIGEN POLYMERSYSTEMEN FÜR MEDIZINISCHE ODER HYGIENISCHE ARTIKEL**

USE OF COLOURED POLYMERIC SYSTEMS FOR MEDICAL OR HYGIENE ARTICLES

UTILISATION DE SYSTÈMES POLYMÈRES COLORÉS POUR DES ARTICLES MÉDICAUX OU HYGIÉNIQUES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **21.02.2006 EP 06110195**

(43) Veröffentlichungstag der Anmeldung:
**12.11.2008 Patentblatt 2008/46**

(73) Patentinhaber: **BASF SE**
**67056 Ludwigshafen (DE)**

(72) Erfinder:
• **LEYRER, Reinhold J**
**67125 Dannstadt-Schauernheim (DE)**
• **WOHLLEBEN, Wendel**
**68161 Mannheim (DE)**
• **ALTMANN, Stephan**
**67146 Deidesheim (DE)**
• **DANIEL, Thomas**
**67165 Waldsee (DE)**

(56) Entgegenhaltungen:
**WO-A-91/15365**

**Beschreibung**

Die Erfindung betrifft die Verwendung von farbigen Polymersystemen mit bei Dehnung veränderbarer Farbe zur Anzeige des Spannungszustandes von am Körper anliegenden hygienischen oder medizinischen Artikeln.

Wässrige Polymerdispersionen sind preiswerte, leicht herstellbare organische Materialien. Aus DE-A 197 17 879 und DE-A 198 20 302 war bekannt, dass sich spezielle Polymerdispersionen zur Herstellung von Polymersystemen aus Polymerteilchen und Matrix eignen und diese Polymersysteme eine Bragg-Reflexion zeigen. Ausführungsformen dieser Polymerdispersionen, bzw. ihrer Verwendung finden sich auch in DE-A 103 21 083, DE-A 103 21 079, DE-A 103 21 084 oder in den am Tag der Einreichung dieser Anmeldung noch nicht veröffentlichten Deutschen Patentanmeldungen mit den Aktenzeichen 10 2005 023 804.1, 10 2005 023 806.8, 10 2005 023 802.5 und 10 2005 023 807.6.

Die Verwendung derartiger Polymersysteme zur Herstellung von optischen Anzeigeelementen (Display) ist in DE-A 102 29 732 beschrieben. In den Anzeigenelementen werden Farbänderungen durch die Änderung der Abstände zwischen den in der Matrix dispergierten Polymerteilchen bewirkt. Ursache der Abstandsänderungen kann z. B. die Einwirkung mechanischer Kräfte oder elektrischer Felder sein.

Aufgabe der vorliegenden Erfindung waren weitere Verwendungen der Polymersysteme.

Demgemäß wurde die eingangs definierte Verwendung gefunden.

Bei dem Polymersystem handelt es sich um ein System aus Polymerteilchen und einem verformbaren Material (Matrix), wobei die Polymerteilchen in der Matrix gemäß einer definierten Raumgitterstruktur verteilt sind.

Zu den Polymerteilchen

Zur Ausbildung einer definierten Raumgitterstruktur sollen die diskreten Polymerteilchen möglichst gleich groß sein. Ein Maß für die Einheitlichkeit der Polymerteilchen ist der sogenannte Polydispersitätsindex, berechnet nach der Formel

$$P.I. = (D90 - D10)/D50$$

worin D90, D10 und D50 Teilchendurchmesser bezeichnen, für die gilt:

D90:90 Gew.% der Gesamtmasse aller Teilchen hat einen Teilchendurchmesser kleiner oder gleich D 90
D50:50 Gew.% der Gesamtmasse aller Teilchen hat einen Teilchendurchmesser kleiner oder gleich D 50
D10:10 Gew.% der Gesamtmasse aller Teilchen hat einen Teilchendurchmesser kleiner oder gleich D 10

Weitere Erläuterungen zum Polydispersitätsindex finden sich z. B. in DE-A 197 17 879 (insbesondere Zeichnungen Seite 1).

Die Teilchengrößenverteilung kann in an sich bekannter Weise, z. B. mit einer analytischen Ultrazentrifuge (W. Mächtle, Makromolekulare Chemie 185 (1984) Seite 1025 - 1039) bestimmt werden und daraus der D 10, D 50 und D 90-Wert entnommen und der Polydispersitätsindex bestimmt werden.

Die Polymerteilchen haben bevorzugt einen D 50-Wert im Bereich um 0,05 bis 5 mm. Es kann sich bei den Polymerteilchen um eine Teilchensorte oder mehrere Teilchensorten mit unterschiedlichem D 50-Wert handeln, wobei jede Teilchensorte einen Polydispersitätsindex bevorzugt kleiner 0,6, besonders bevorzugt kleiner 0,4 und ganz besonders bevorzugt kleiner 0,3 und insbesondere kleiner 0,15 hat.

Insbesondere bestehen die Polymerteilchen nun aus einer einzigen Teilchensorte. Der D 50-Wert liegt dann vorzugsweise zwischen 0,05 und 2 mm, besonders bevorzugt liegt er zwischen 100 und 400 Nanometer.

Auch Polymerteilchen, welche z. B. aus 2 oder 3, vorzugsweise 2 hinsichtlich des D 50-Wertes unterschiedlichen Teilchensorten bestehen, können eine gemeinsame Gitterstruktur bilden (kristallisieren) sofern für jede Teilchensorte die obige Bedingung hinsichtlich des Polydispersitätsindexes erfüllt ist. Geeignet sind z. B. Gemische in Teilchensorten mit einem D 50-Wert von 0,3 bis 0,5 mm und mit einem D 50-Wert von 0,1 bis 0,3 mm.

Die Polymerteilchen bestehen vorzugsweise aus einem Polymer mit einer Glasüberganstemperatur größer 30 °C, besonders bevorzugt größer 50 °C und ganz besonders bevorzugt größer 70 °C, insbesondere größer 90 °C.

Die Glasübergangstemperatur lässt sich nach üblichen Methoden wie Differentialthermoanalyse oder Differentail Scanning Calorimetrie (s. z. B. ASTM 3418/82, sog. "midpoint temperature") bestimmen.

Das Polymer besteht vorzugsweise zu mindestens 40 Gew.-%, bevorzugt zu mindestens 60 Gew.-%, besonders bevorzugt zu mindestens 80 Gew.-% aus sogenannten Hauptmonomeren.

Die Hauptmonomeren sind ausgewählt aus C1-C20-Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden, Vinylethern von 1 bis 10 C-Atome enthaltenden Alkoholen, aliphatischen Kohlenwasserstoffen mit 2 bis 8 C-Atomen und 1 oder 2 Doppelbindungen oder Mischungen dieser Monomeren.

Zu nennen sind z. B. (Meth)acrylsäurealkylester mit einem C1-C10-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z. B. Vinyllaurat, -stearat, Vinylpropionat, Versaticsäurevinylester und Vinylacetat.

Als vinylaromatische Verbindungen kommen Vinyltoluol, a- und p-Methylstyrol, alpha-Butylstyrol, 4-n-Butylstyrol, 4-n-Decylstyrol und vorzugsweise Styrol in Betracht. Beispiele für Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als Vinylether zu nennen sind z. B. Vinylmethylether oder Vinylisobutylether. Bevorzugt wird Vinylether von 1 bis 4 C-Atome enthaltenden Alkoholen.

Als Kohlenwasserstoffe mit 2 bis 8 C-Atomen und ein oder zwei olefinischen Doppelbindungen seien Butadien, Isopren und Chloropren genannt, mit einer Doppelbindung z. B. Ethylen oder Propylen.

Als Hauptmonomere bevorzugt sind die C1- bis C20-Alkylacrylate und -methacrylate, insbesondere C1- bis C8-Alkylacrylate und -methacrylate, Vinylaromaten, insbesondere Styrol, und deren Mischungen, insbesondere auch Mischungen der Alkyl(meth)acrylate und Vinylaromaten.

Ganz besonders bevorzugt sind Methylacrylat, Methylmethacrylat, Ethylacrylat, n-Butylacrylat, n-Hexylacrylat, Octylacrylat und 2-Etyhlhexylacrylat, Styrol sowie Mischungen dieser Monomere.

Die Polymerteilchen sind vorzugsweise chemisch vernetzt. Dazu können Monomere mit mindestens zwei polymerisierbaren Gruppen, z. B. Divinylbenzol oder Allylmethacrylat, mitverwendet erden (interne Vernetzung). Es können aber auch Vernetzer zugesetzt werden (externe Vernetzung).

Zur Matrix

Zwischen der Matrix und den Polymeren sollte ein Unterschied im Brechungsindex bestehen.

Bevorzugt sollte der Unterschied mindestens 0,01, besonders bevorzugt mindestens 0,1 betragen.

Dabei kann sowohl die Matrix als auch das Polymer den höheren Brechungsindex haben. Entscheidend ist, dass ein Unterschied besteht.

Die Matrix besteht aus einem verformbaren Material. Unter Verformbarkeit wird verstanden, dass die Matrix eine räumliche Verschiebung der diskreten Polymerteilchen bei Anwendung äußerer Kräfte (z. B. mechanisch, elektromagnetisch) erlaubt.

Bevorzugt besteht daher die Matrix aus einem organischen Material, bzw. organischen Verbindungen mit einem Schmelzpunkt oder eine Glasüberganstemperatur unterhalb 20 °C, besonders bevorzugt unterhalb 10 °C, ganz besonders bevorzugt unterhalb 0 °C (bei 1 bar).

Es kommen auch organische Verbindungen mit einem Schmelzpunkt oder einer Glasübergangstemperatur (Tg) oberhalb 20°C in Betracht, hierbei ist jedoch ein zwischenzeitliches Aufheizen oberhalb des Schmelzpunktes oder der Tg erforderlich, wenn die Abstände der Polymerteilchen geändert werden sollen (siehe unten).

In Betracht kommen Flüssigkeiten wie Wasser oder höher viskose Flüssigkeiten wie Glycerin oder Glycol.

Bevorzugt sind polymere Verbindungen, z. B. Polykondensate, Polyaddukte oder durch radikalische Polymerisation erhältlich Polymere.

Genannt seien z. B. Polyester, Polyamide, Formaldehydharze, wie Melamin-, Harnstoff- oder Phenol-Formaldehydkondensate, Polyepoxide, Polyurethane, oder auch die oben genannten Polymere, welche die aufgeführten Hauptmonomere enthalten, z. B. Polyacrylate, Polybutadiene, Styrol-/Butadiencopolymerisate.

Zur Herstellung

Herstellungsmethoden sind in DE-A 197 17 879 und DE-A 198 20 302 beschrieben.

Herstellung der diskreten Polymerteilchen.

Die Herstellung der Polymerteilchen, bzw. Polymeren erfolgt in einer bevorzugten Ausführungsform durch Emulsionspolymerisation, es handelt sich daher um ein Emulsionspolymerisat.

Die Emulsionspolymerisation ist insbesondere bevorzugt, weil so Polymerteilchen mit gleichmäßiger Kugelform erhältlich sind.

Die Herstellung kann jedoch z. B. auch durch Lösungspolymerisation und anschließende Dispergierung in Wasser erfolgen.

Bei der Emulsionspolymerisation werden ionische und/oder nicht-ionische Emulgatoren und/oder Schutzkolloide bzw. Stabilisatoren als grenzflächenaktive Verbindungen verwendet.

Eine ausführliche Beschreibung geeigneter Schutzkolloide findet sich in Houben-Weyl, Methoden der organischen Chemie, Band XIV/1, Makromolekulare Stoffe, Georg-Thieme-Verlag, Stuttgart, 1961, S. 411 bis 420. Als Emulgatoren kommen sowohl anionische, kationische als auch nichtionische Emulgatoren in Betracht. Vorzugsweise werden als grenzflächenaktive Substanzen Emulgatoren eingesetzt, deren Molekülärgewicht im Unterschied zu den Schutzkolloiden üblicherweise unter 2000 g/mol liegen.

Die grenzflächenaktive Substanz wird üblicherweise in Mengen von 0,1 bis 10 Gew.-%, bezogen auf die zu polymerisierenden Monomeren verwendet.

Wasserlösliche Initiatoren für die Emulsionspolymerisation sind z. B. Ammonium- und Alkalimetallsalze der Peroxidischwefelsäure, z. B. Natriumperoxodisulfat, Wasserstoffperoxid oder organische Peroxide, z. B. tert-Butylhydroperoxid.

Geeignet sind auch sogenannte Reduktions-Oxidations(Red-Ox)-4nitiator Systeme.

Die Red-Ox-Initiator-Systeme bestehen aus mindestens einem meist anorganischen Reduktionsmittel und einem anorganischen oder organischen Oxidationsmittel.

Bei der Oxidationskomponente handelt es sich z. B. um die bereits vorstehend genannten Initiatoren für die Emulsionspolymerisation.

Bei der Reduktionskomponenten handelt es sich z. B. um Alkalimetallsalze der schwefligen Säure, wie z. B. Natriumsulfit, Natriumhydrogensulfit, Alkalisalze der Dischwefligen Säure wie Natriumdisulfit, Bisulfitadditionsverbindungen aliphatischer Aldehyde und Ketone, wie Acetonbisulfit oder Reduktionsmittel wie Hydroxymethansulfinsäure und deren Salze, oder Ascorbinsäure. Die Red-Ox-Initiator-Systeme können unter Mitverwendung löslicher Metallverbindungen, deren metallische Komponente in mehreren Wertigkeitsstufen auftreten kann, verwendet werden.

Übliche Red-Ox-Initiator-Systeme sind z. B. Ascorbinsäure/Eisen(11)sulfat/Natriumperoxidisulfat, tert-Butylhydroperoxid/Natriumdisulfit, tert-Butylhydroperoxid/Na-Hydroxymethansulfinsäure. Die einzelnen Komponenten, z. B. die Reduktionskomponente, können auch Mischungen sein z. B. eine Mischung aus dem Natriumsalz der Hydroxymethansulfinsäure und Natriumdisulfit.

Die Menge der Initiatoren beträgt im allgemeinen 0,1 bis 10 Gew.-%, bevorzugt 0,5 bis 5 Gew.-%, bezogen auf die zu polymerisierenden Monomeren. Es können auch mehrere, verschiedene Initiatoren bei der Emulsionspolymerisation Verwendung finden.

Die Emulsionspolymerisation erfolgt in der Regel bei 30 bis 130, vorzugsweise 50 bis 90 °C. Das Polymerisationsmedium kann sowohl nur aus Wasser, als auch aus Mischungen aus Wasser und damit mischbaren Flüssigkeiten wie Methanol bestehen. Vorzugsweise wird nur Wasser verwendet. Die Emulsionspolymerisation kann sowohl als Batchprozess als auch in Form eines Zulaufverfahrens, einschließlich Stufen- oder Gradientenfahrweise, durchgeführt werden. Bevorzugt ist das Zulaufverfahren, bei dem man einen Teil des Polymerisationsansatzes vorlegt, auf die Polymerisationstemperatur erhitzt, anpolymerisiert und anschließend den Rest des Polymerisationsansatzes, üblicherweise über mehrere räumlich getrennte Zuläufe, von denen einer oder mehrere die Monomeren in reiner oder in emulgierter Form enthalten, kontinuierlich, stufenweise oder unter Überlagerung eines Konzentrationsgefälles unter Aufrechterhaltung der Polymerisation der Polymerisationszone zuführt. Bei der Polymerisation kann auch z. B. zur besseren Einstellung der Teilchengröße eine Polymersaat vorgelegt werden.

Die Art und Weise, in der der Initiator im Verlauf der radikalischen wässrigen Emulsionspolymerisation dem Polymerisationsgefäß zugegeben wird, ist dem Durchschnittsfachmann bekannt. Es kann sowohl vollständig in das Polymerisationsgefäß vorgelegt, als auch nach Maßgabe seines Verbrauchs im Verlauf der radikalischen wässrigen Emulsionspolymerisation kontinuierlich oder stufenweise eingesetzt werden. Im einzelnen hängt dies von der chemischen Natur des Initiatorsystems als auch von der Polymerisationstemperatur ab. Vorzugsweise wird ein Teil vorgelegt und der Rest nach Maßgabe des Verbrauchs der Polymerisationszone zugeführt.

Eine einheitliche Teilchengrößenverteilung, d.h. ein geringer Polydispersitätsindex ist durch den Fachmann bekannte Maßnahmen erhältlich, z. B. durch Variation der Menge der grenzflächenaktiven Verbindung (Emulgator oder Schutzkolloide) und/oder entsprechende Rührergeschwindigkeiten.

Zur Entfernung der Restmonomeren wird üblicherweise auch nach dem Ende der eigentlichen Emulsionspolymerisation, d.h. nach einem Umsatz der Monomeren von mindestens 95 %, Initiator zugesetzt.

Die einzelnen Komponenten können dem Reaktor beim Zulaufverfahren von oben, in der Seite oder von unten durch den Reaktorboden zugegeben werden.

Bei der Emulsionspolymerisation werden wässrige Dispersionen des Polymeren in der Regel mit Feststoffgehalten von 15 bis 75 Gew.-%, bevorzugt von 40 bis 75 Gew.-% erhalten.

Herstellung der Mischung Polymerteilchen / Matrix (Schicht)

Wasser oder Lösemittel als Matrix

Bei der Emulsionspolymerisation wird direkt eine wässrige Dispersion der Polymerteilchen erhalten. Das Wasser kann einfach soweit entfernt werden bis sich die Gitterstruktur der Polymerteilchen, erkennbar an den dabei zu beobachtenden farblichen Effekten, einstellt.

Sind andere Lösemittel gewünscht, kann Wasser in einfacher Weise gegen diese Lösemittel ausgetauscht werden.

Polymere Verbindungen als Matrix

Die bei der Emulsionspolymerisation erhaltene wässrige Dispersion der diskreten Polymerteilchen kann mit der zur Einstellung der Gitterstruktur benötigten Menge der polymeren Verbindung gemischt und anschließend das Wasser entfernt werden. Aufgrund der oftmals hohen Viskosität der polymeren Verbindung kann es vorteilhaft sein, die Polymerteilchen zunächst mit den Aufbaukomponenten der polymeren Verbindung zu mischen und dann, nach erfolgter Dispergierung der Polymerteilchen, diese Aufbaukomponenten z. B. durch Kondensation, Adduktbildung zu den polymeren Verbindungen umzusetzen.

Emulsionspolymerisate als diskrete Polymerteilchen und Emulsionspolymerisate als Matrix

Bevorzugt sind Emulsionspolymerisate als diskrete Polymerteilchen, und Emulsionspolymerisate als Matrix

Die entsprechenden Emulsionspolymerisate können einfach gemischt und anschließend das Wasser entfernt werden. Soweit die Emulsionspolymerisate für die Matrix eine Glasübergangstemperatur unter 20 °C haben (siehe oben), verfilmen die Polymerteilchen bei Raumtemperatur und bilden die durchgehende Matrix, bei höherer Tg ist eine Erwärmung auf Temperaturen oberhalb der Tg erforderlich.

Besonders einfach und vorteilhaft ist es, beide Emulsionspolymerisate in einem Schritt als Kern/Schale-Polymerisat herzustellen. Dazu wird die Emulsionspolymerisation 2-stufig durchgeführt. Zunächst werden die Monomeren polymerisiert, welche den Kern (= spätere diskrete Polymerteilchen) bilden, dann werden in einer 2. Stufe in Gegenwart des Kerns die Monomeren polymerisiert, welche die Schale (= spätere Matrix) bilden.

Bei der späteren Entfernung des Wassers verfilmt die weiche Schale, deren Glasübergangstemperatur unter 20 °C liegt und die verbleibenden (harten) Kerne sind als diskrete Polymerteilchen in der Matrix verteilt.

Bei den Polymerteilchen handelt es sich daher besonders bevorzugt um den Kern von Kern/Schale Polymeren, die Matrix wird durch Verfilmung der Schale gebildet.

Der Abstand zwischen den Polymerteilchen beträgt vorzugsweise 100 bis 400 Nanometer, so dass elektromagnetische Strahlung im Bereich des sichtbaren Lichts reflektiert wird (Bragg-Reflektion).

Kern/Schale Polymerisate, erhältlich durch Emulsionspolymerisation sind im Rahmen der vorliegenden Erfindung besonders bevorzugt.

Besonders geeignete Ausführungsformen zu den Kern/Schale - Emulsionspolymerisaten finden sich in DE-A 197 17 879, DE-A 198 20 302, DE-A 103 21 083, DE-A 103 21 079, DE-A 103 21 084 oder in den am Tag der Einreichung dieser Anmeldung noch nicht veröffentlichten Deutschen Patentanmeldungen mit den Aktenzeichen 10 2005 023 804.1, 10 2005 023 806.8, 10 2005 023 802.5 und 10 2005 023 807.6.

Die polymeren Verbindungen können auch vernetzt werden, so dass sie elastische Eigenschaften haben. Soweit Vernetzung gewünscht ist, erfolgt sie vorzugsweise bei oder nach der Verfilmung, zum Beispiel durch thermisch oder photochemisch ausgelöste Vernetzungsreaktion eines Vernetzers, der zugesetzt wird oder bereits an das Polymer gebunden sein kann.

Die Vernetzung der Matrix bewirkt eine Rückstellkraft, welche auf die diskreten Polymerteilchen wirkt. Ohne Einwirkung äußerer Kräfte nehmen die Polymerteilchen dann wieder die vorgegebene Ausgangsposition ein.

Zum Aufbau des Polymersystems aus Polymerteilchen und Matrix

Das Polymersystem verursacht einen optischen Effekt, das heißt eine zu beobachtende Reflexion durch Interferenz des an den Polymerteilchen gestreuten Lichts.

Die Wellenlänge der Reflexion kann dabei je nach Abstand der Polymerteilchen im gesamten elektromagnetischen Spektrum liegen. Vorzugsweise liegt die Wellenlänge im UV-Bereich, IR-Bereich und insbesondere im Bereich des sichtbaren Lichts.

Die Wellenlänge der zu beobachtenden Reflexion hängt dabei nach der bekannten Bragg-Gleichung von dem Netzebenenabstand, hier der Abstand zwischen den in einer Raumgitterstruktur in der Matrix angeordneten Polymerteilchen, ab.

Damit sich die gewünschte Raumgitterstruktur mit dem gewünschten Abstand zwischen den Polymerteilchen einstellt, ist insbesondere der Gewichtsanteil der Matrix entsprechend zu wählen. Bei den vorstehend beschriebenen Herstellmethoden sollten die organischen Verbindungen, z. B. polymere Verbindungen in entsprechender Menge eingesetzt werden.

Der Gewichtsanteil der Matrix wird insbesondere so bemessen, dass eine Raumgitterstruktur der Polymerteilchen entsteht, welche elektromagnetische Strahlung im gewünschten Bereich reflektiert.

Der Abstand zwischen den Polymerteilchen (jeweils bis zum Mittelpunkt der Teilchen) beträgt geeigneterweise 100 bis 400 nm, wenn ein Farbeffekt, d.h. eine Reflexion im Bereich des sichtbaren Lichts gewünscht ist.

Zur Verwendung

Erfindungsgemäß werden die farbigen Polymersysteme zur Anzeige des Spannungszustandes von am Körper anliegenden hygienischen oder medizinischen Artikeln verwendet. Bei Dehnung ändert sich die Farbe des Polymersystems. Durch die entsprechende Farbänderung kann daher festegestellt werden, ob ein Artikel zu stark gedehnt ist, d. h. zu eng anliegt und so zu Verletzungen am menschlichen oder tierischen Körper oder sonstigen Beeinträchtigungen des Wohlbefindens führen kann.

Bei den medizinischen Artikeln handelt es sich z. B. um Pflaster oder Verschlüsse für Verbände.

Bei den hygienischen Artikeln handelt es sich z. B. um Windeln oder Inkontinenzartikel.

Die hygienischen oder medizinischen Artikel können ganz oder stellenweise mit dem Polymersystem beschichtet oder imprägniert sein. Es reicht aus, dass ein gut sichtbarer Bereich, der bei der Anwendung des Artikels gedehnt wird, entsprechend beschichtet oder imprägniert ist.

Insbesondere kann das Polymersystem auch auf Trägern, z. B. Klebebändern oder Etiketten, beschichtet sein. Die Träger sollten eine ausreichende Dehnbarkeit haben und so eine Farbänderung des Polymersystems durch Dehnung ermöglichen.

Die beschichteten Träger können für medizinische oder hygienische Artikel verwendet werden. Die Träger können an den medizinischen oder hygienischen Artikeln an den Stellen, die sich der Anwendung der Artikel dehnen, angebracht werden. Die Träger können insbesondere gleichzeitig weitere Funktionen haben, insbesondere können sie zum Verschließen oder Befestigen der medizinischen oder hygienischen Artikel verwendet werden.

Bei Dehnung des medizinischen oder hygienischen Artikels, bzw. von Teilen des Artikels, insbesondere den Verschlussteilen ändert sich die Farbe des mitgedehnten Polymersystems. Die Art der Farbänderung zeigt das Ausmaß der Dehnung an.

Es ist daher unmittelbar zu erkennen, ob medizinische oder hygienische Artikel zu eng anliegen und das Wohlbefinden beeinträchtigen.

Beispiele

Herstellung der Polymeren

Die folgenden Ausführungsbeispiele veranschaulichen die Erfindung. Die in den Beispielen benutzten Emulgatoren haben folgende Zusammensetzungen:

Emulgator 1:30 Gew.-%ige Lösung des Natriumsalzes eines ethoxylierten und sulfatierten Nonylphenols mit ca. 25 mol/mol Ethylenoxyd-Einheiten.

Emulgator 2:40 Gew.-%ige Lösung eines Natriumsalzes eines C12/C14-Paraffinsulfonates.

Emulgator 3:15 Gew.-%ige Lösung von linearem Natriumdodecylbenzolsulfonat.

Die Teilchengrößen-Verteilungen wurden mit Hilfe einer analytischen Ultrazentrifuge oder mit Hilfe der kapillarhydrodynamischen Fraktionierungsmethode (CHDF 1100 Particle Size Analyzer der Fa. Matec Applied Sciences) bestimmt, und aus den erhaltenen Werten der P.I.- Wert nach der hier angegebenen Formel

$$P.I. = (D90 - D10)/D50$$

berechnet. Lösungen sind, soweit nichts Anderes angegeben ist, wässrige Lösungen.

Die in den Beispielen verwendete Angabe pphm bedeutet Gew.-Teile bezogen auf 100 Gew.-Teile Gesamtmonomere.

Die für Monomere verwendeten Abkürzungen haben folgende Bedeutungen: AS = Acrylsäure, n-BA = n-Butylacrylat, DVB = Divinylbenzol, EA = Ethylacrylat, MAS = Methacrylsäure, MAMol = N-Methylolmethacrylamid, NaPS = Natriumpersulfat.

Beispiel 1: Herstellung eines Emulsionspolmerisats

In einem mit Ankerrührer, Thermometer, Gaseinleitungsrohr, Tropftrichter und Rückflusskühler versehenen Glasreaktor wird eine Dispersion von 0,9 g (0,20 pphm) Polystyrol-Saat (Partikelgröße: 30 nm) in 500 ml Wasser vorgelegt und unter Rühren in einem Heizbad erwärmt, wobei gleichzeitig die Luft durch Einleiten von Stickstoff verdrängt wird. Wenn das Heizbad die voreingestellte Temperatur von 85 C und der Reaktorinhalt die Temperatur von 80 C erreicht hat, wird die Stickstoffeinleitung unterbrochen und es werden gleichzeitig im Verlauf von 3 Stunden eine Emulsion von 445,5 g Styrol (99.0 Gew.-%), 4,5 g Divinylbenzol (1.0 Gew.-%) und 14,5 g Emulgator 1 (1,0 pphm) in 501,3 ml Wasser und 54,0 g einer 2,5 gew.- %igen wässrigen Lösung von Natriumpersulfat (0,3 pphm) zutropfen lassen. Nach vollständigem Zulauf der Lösungen wird die Polymerisation noch 7 Stunden bei 85 C fortgesetzt und anschließend auf Zimmertemperatur abgekühlt.

Die Dispersion hat folgende Eigenschaften:

| | |
|---|---|
| Feststoffgehalt: | 29,6 Gew.-% |
| Partikelgröße: | 255 nm |
| Koagulatanteil: | < l g |
| pH-Wert: | 2.3 |
| Polydispersitätsindex: | 0,13 |
| Brechungsindex: | 1,59 |

Dieses Beispiel wurde mehrfach wiederholt, wobei die Konzentration der Saatpartikel variiert wurde. Die folgende Tabelle I gibt eine Übersicht über die erhaltenen Versuchsergebnisse.

Tabelle I

| Beisp. Nummer | 1A | 1B | 1C | 1D | 1E | 1F | IG |
|---|---|---|---|---|---|---|---|
| Saat-Konz. Gew.%. | 0,20 | 0,15 | 0,10 | 0,053 | 0,30 | 0,53 | 3,16 |
| Feststoff Gehalt Gew.% | 28,8 | 28,4 | 28,5 | 29,4 | 29,3 | 30,0 | 28,6 |
| Part.-Größe [nm] | 256 | 280 | 317 | 357 | 222 | 188 | 125 |
| P.I. | 0,13 | - | - | 0,19 | - | - | 0,221 |

Beispiel 2: Herstellung eines Emulsionspolymerisats mit Kern/Schale-Aufbau.

In einem mit Ankerrührer, Thermometer, Gaseinleitungsrohr, Tropftrichter und Rückflusskühler versehenen Glasreaktor werden 300 g der in Beispiel 1 A erhaltenen Dispersion von Kernpartikeln vorgelegt und unter Rühren in einem Heizbad erwärmt, wobei gleichzeitig die Luft durch Einleiten von Stickstoff verdrängt wird.

Wenn das Heizbad die voreingestellte Temperatur von 85 °C und der Reaktorinhalt die Temperatur von 80°C erreicht hat, wird die Stickstoffeinleitung unterbrochen und es werden gleichzeitig im Verlauf von 1,5 Stunden.

a) eine Mischung von 84,7 g (98,0 Gew.-%) n-Butylacrylat, 0,86 (1,0-Gew%) Acrylsäure, 5,76 g (1,0 Gew.-%) einer 15 gew.-%igen Lösung von N-Methylolmethacrylamid, 2,86 g einer 31 gew.-%igen Lösung (0,97 pphm) des Emulgators I und 12.4 g Wasser sowie

b) 17,3 g einer 2,5 gew.-%igen wäßrigen Lösung von Natriumpersulfat (0,5 pphm) zugetropft.

Nach vollständigem Zulauf der Lösungen wird die Polymerisation noch 3 Stunden bei 85 C fortgesetzt. Danach wird die erhaltene Dispersion von Kern/Schale-Partikeln auf Zimmertemperatur abgekühlt.

Die Dispersion hat folgende Eigenschaften:

| | |
|---|---|
| Feststoffgehalt: | 40,6 Gew.-% |
| Partikelgröße: | 307 nm |
| Polydispersitätsindex (PI): | 0,16 |

(fortgesetzt)

| | |
|---|---|
| Gewichtsverhältnis Kern:Schale: | 1:1 (berechnet) |
| Brechungsindex des Schalenpolymers-. | 1,44 |

Dieses Beispiel wurde noch zweimal wiederholt, wobei die Konzentration der Kernpartikel und das Gewichtsverhältnis von Kern/Schale variiert wurde. Die folgende Tabelle 2 gibt eine Übersicht über die erhaltenen Versuchsergebnisse.

Tabelle 2

| Beisp. Nummer | 2A | 2B | 2C |
|---|---|---|---|
| Kernanteil (Gew.-Teile) | 100,0 | 133,3 | 225,0 |
| n-BA [Gew.-%] | 98,0 | 98,0 | 98,0 |
| AS (Gew.-%) | 1,0 | 1,0 | 1,0 |
| MAMol [Gew.-%] | 1,0 | 1,0 | 1,0 |
| Schale:Kern-Verhältnis | 1:1 | 0,75:1 | 0,44:1 |
| Partikelgröße [nml | 301 | 308 | 284 |
| P.I. | 0,162 | 0,137 | 0,144 |
| Feststoffgchalt [Gew.-%] | 39,4 | 40,6 | 35,2 |
| Gew.-% für nBA, AS und MAMol sind auf die Schale bezogen. | | | |

Herstellung einer reflektierenden Schicht

Beispiel 3A

15 g der gemäß Beispiel 2A erhaltenen Dispersion werden in einer Silicongummi-Schale bei Zimmertemperatur getrocknet. Man erhält eine leuchtend effektfarbige Schicht von gummiartiger Elastizität. Wird diese in einem Vakuumtrockenschrank 1 Stunde bei 120°C gehalten und anschließend auf Zimmertemperatur abgekühlt, so erhöht sich ihre Elastizität noch und es zeigt sich eine leichte Veränderung der Farbe. Beim Dehnen der Schicht ändert sich ihre Farbe mit dem Dehnungsverhältnis von Braun über Grün nach Violett.

Beispiel 3B

135 g der gemäß Beispiel 2A erhaltenen Dispersion werden mit 15 g einer feinteiligen, 20 gew.-%igen wässrigen Dispersion eines Copolymerisats von 94 Gew.-% Ethylacrylat und 6 Gew.-% Methacrylsäure mit einer mittleren Teilchengröße von 30 nm und einer Glastemperatur von ca. 0 C gemischt und die Mischung in einer Silicongummi-Schale bei Zimmertemperatur getrocknet. Man erhält eine effektfarbige Schicht, die mechanisch noch stabiler ist als die in Beispiel 3A erhaltene. Das Beispiel veranschaulicht die Erleichterung und Verbesserung der Verfilmung durch den Copolymerzusatz.

Beispiel 3C

20 g der gemäß Beispiel 2A erhaltenen Dispersion werden mit 2 g Diethylenglycoldiethylether (DGDE), gemischt, mit 10 g Wasser verdünnt und die Mischung in einer Silicongummi-Schale bei Zimmertemperatur getrocknet. Man erhält eine leuchtend effektfarbige Schicht, die mechanisch noch stabiler ist als die in Beispiel 3A erhaltene. Das Beispiel zeigt, dass auch der Zusatz des DGDE das Verfilmen der Schalepolymeren bereits bei Zimmertemperatur ermöglicht, dabei aber nur einen geringen Einfluss auf die Farbe der verfilmten Schicht hat.

Beispiel 4

15 g der gemäß Beispiel 2C erhaltenen Dispersion werden in einer Silicongummi-Schale bei Zimmertemperatur durch Verdunstung des Wassers getrocknet. Anschließend wird die erhaltene leuchtend effektfarbige Schicht, in einem Vakuumtrockenschrank 1 Stunde bei 120°C gehalten und anschließend auf Zimmertemperatur abgekühlt. Es wird ein harter, mechanisch stabiler, transparenter Film erhalten, der eine leuchtende, mit dem Beleuchtungs- und Betrachtungswinkel veränderliche Farbe aufweist. Durch Zugabe einer feinteiligen weichen Dispersion analog Beispiel 3B und/oder durch Zugabe von Weichmachern analog Beispiel 3C lässt sich die Härte der Schicht nach Bedarf reduzieren, so dass bei Dehnung der Schichten oder beim Pressen der Schichten Farbveränderungen auftreten analog dem Beispiel 3A.

Beispiel 5

Wird in Beispiel 2 A die Teilchengröße der eingesetzten Saat verändert, d.h. an Stelle der Saat 1A beispielsweise die Saat 1 D verwendet, so verschiebt sich der Farbeindruck der analog Beispiel 3A hergestellten Filme in den länger welligen Bereich des Farbspektrums. Entsprechend wird durch Einsatz eines kleineren Saatpartikels wie beispielsweise Saat 1 G der Farbeindruck der analog Beispiel 3 A erhaltenen Schichten in den kürzer welligen Bereich des Farbspektrums hin verschoben.

**Patentansprüche**

1. Verwendung von farbigen Polymersystemen mit bei Dehnung veränderbarer Farbe zur Anzeige des Spannungszustandes von am Körper anliegenden hygienischen oder medizinischen Artikeln, **dadurch gekennzeichnet, dass** es sich bei dem Polymersystem um ein System aus Polymerteilchen und einem verformbaren Material (Matrix) handelt, wobei die Polymerteilchen in der Matrix gemäß einer definierten Raumgitterstruktur verteilt sind.

2. Verwendung gemäß Anspruch 1, **dadurch gekennzeichnet, dass** es sich bei den Polymerteilchen um eine oder mehrere Teilchensorten mit einem mittleren Teilchendurchmesser im Bereich von 0,05 bis 5 $\mu$m handelt, wobei jede Teilchensorte einen Polydispersitätsindex (PI) kleiner 0,6 hat, berechnet nach der Formel

$$\text{P.I.} = (D90 - D10)/D50$$

worin D90, D10 und D50 Teilchendurchmesser bezeichnen, für die gilt:

    D 90: 90 Gew % der Gesamtmasse aller Teilchen hat einen Teilchendurchmesser kleiner oder gleich D 90
    D 50: 50 Gew % der Gesamtmasse aller Teilchen hat einen Teilchendurchmesser kleiner oder gleich D 50
    D10: 10 Gew % der Gesamtmasse aller Teilchen hat einen Teilchendurchmesser kleiner oder gleich D 10

3. Verwendung gemäß einem der Ansprüche 1 bis 2, **dadurch gekennzeichnet, dass** die diskreten Polymerteilchen eine Glasübergangstemperatur größer 30 °C haben.

4. Verwendung gemäß einem der Ansprüche 1 bis 3, **dadurch gekennzeichnet, dass** sich die Polymerteilchen und die Matrix im Brechungsindex unterscheiden.

5. Verwendung gemäß einem der Ansprüche 1 bis 4, **dadurch gekennzeichnet, dass** auch die Matrix aus einer polymeren Verbindung besteht.

6. Verwendung gemäß einem der Ansprüche 1 bis 5, **dadurch gekennzeichnet, dass** es sich bei den Polymerteilchen um den Kern von Kern/Schale Polymeren handelt und die Matrix durch Verfilmung der Schale gebildet wird.

7. Verwendung gemäß einem der Ansprüche 1 bis 6, **dadurch gekennzeichnet, dass** der Abstand zwischen den Polymerteilchen 100 bis 400 Nanometer beträgt, so dass elektromagnetische Strahlung im Bereich des sichtbaren Lichts reflektiert wird.

8. Verwendung gemäß einem der Ansprüche 1 bis 7, **dadurch gekennzeichnet, dass** die hygienischen oder medizinischen Artikel ganz oder stellenweise mit dem Polymersystem beschichtet oder imprägniert sind.

9. Verwendung gemäß einem der Ansprüche 1 bis 8, **dadurch gekennzeichnet, dass** das Polymersystem auf Trägern, z. B. Klebebändern oder Etiketten, beschichtet ist und die beschichteten Träger für die medizinischen oder hygienischen Artikel verwendet werden.

## Claims

1.  The use of colored polymer systems having a color which is changeable in the case of a strain and is intended for indicating the stress state of hygiene or medical articles adjacent to the body, wherein the polymer system is a system comprising polymer particles and a deformable material (matrix), the polymer particles being distributed in the matrix according to a defined space lattice structure.

2.  The use according to claim 1, wherein the polymer particles are one or more particle types having a median particle diameter in the range from 0.05 to 5 $\mu$m, each particle type having a polydispersity index (PI) of less than 0.6, calculated according to the formula

    $$P.I. = (D90-D10)/D50$$

    where D90, D10 and D50 are particle diameters for which the following applies:

    D 90: 90% by weight of the total mass of all particles have a particle diameter of less than or equal to D 90
    D 50: 50% by weight of the total mass of all particles have a particle diameter of less than or equal to D 50
    D 10: 10% by weight of the total mass of all particles have a particle diameter of less than or equal to D 10.

3.  The use according to either of claims 1 and 2, wherein the discrete polymer particles have a glass transition temperature greater than 30°C.

4.  The use according to any of claims 1 to 3, wherein the polymer particles and the matrix differ in the refractive index.

5.  The use according to any of claims 1 to 4, wherein the matrix too consists of a polymeric compound.

6.  The use according to any of claims 1 to 5, wherein the polymer particles are the core of core/shell polymers and the matrix is formed by film formation of the shell.

7.  The use according to any of claims 1 to 6, wherein the spacing between the polymer particles is from 100 to 400 nanometers so that electromagnetic radilation in the range of visible light is deflected.

8.  The use according to any of claims 1 to 7, wherein the hygiene or medical articles are completely or partly coated or impregnates with the polymer system.

9.  The use according to any of claims 1 to 8, wherein the polymer system is applied to substrates, e.g. adhesive tapes or labels, by coating, and the coated substrates are used for medical or hygiene articles.

## Revendications

1.  Utilisation de systèmes polymères colorés avec une couleur modifiable lors de l'élongation pour indiquer l'état de tension d'objets hygiéniques ou médicaux placés sur le corps, **caractérisée en ce qu'**il s'agit, pour le système polymère, d'un système de particules polymère et d'un matériau déformable (matrice), les particules polymères étant réparties dans la matrice selon une structure de réseau spatial définie.

2.  Utilisation selon la revendication 1, **caractérisée en ce qu'**il s'agit, pour les particule polymères, d'un ou de plusieurs types de particules présentant un diamètre moyen des particules dans la plage de 0,05 à 5 $\mu$m, chaque type de particules présentant un indice de polydispersité (PI) inférieur à 0,6, calculé selon la formule

    $$P.I. = (D90-D10)/D50$$

    dans laquelle D90, D10 et D50 désignent les diamètres des particules, où

- D90 : 90% en poids de la masse totale de toutes les particules présentant un diamètre de particule intérieur ou égal à D90
- D50 : 50% en poids de la masse totale de toutes les particules présentant un diamètre de particule inférieur ou égal à D50
- D10 : 10% en poids de la masse totale de toutes les particules présentent un diamètre de particule intérieur ou égal à D10.

3. Utilisation selon l'une quelconque des revendications 1 à 2, **caractérisée en ce que** les particules polymères discrètes présentent une température de transition vitreuse supérieure à 30°C.

4. Utilisation selon l'une quelconque des revendications 1 à 3, **caractérisée en ce que** les particule polymères et la matrice se distinguent en ce qui concerne l'indice de réfraction.

5. Utilisation selon l'une quelconque des revendications 1 à 4, **caractérisée en ce que** à motrice est également constituée par un composé polymère.

6. utilisation selon l'une quelconque des revendications 1 à 5, **caractérisée en ce qu'**il s'agit, pour les particules polymères, du noyau de polymères à noyau/coquille et la matrice est formée par transformation en film de la coquille.

7. Utilisation selon l'une quelconque des revendications 1 à 6, **caractérisée en ce que** la distance entre les particules polymères est de 100 à 400 nanomètres, de manière telle que le rayonnement électromagnétique est réfléchi dans la plage de la lumière visible.

8. Utilisation selon l'une quelconque des revendications 1 à 7, **caractérisée en ce que** les objets hygiéniques ou médicaux sont revêtus ou imprégnés totalement ou par endroits par le système polymère.

9. Utilisation selon l'une quelconque des revendications 1 à 8, **caractérisée en ce que** le système polymère est revêtu sur des supports, par exemple des bondes adhésives ou des étiquettes et les supports revêtus sont utilisés pour les objets médicaux ou hygiéniques.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19717879 A
- DE 19820302 A
- DE 10321083 A
- DE 10321079 A
- DE 10321084 A
- DE 102005023804
- DE 102005023806
- DE 102005023802
- DE 102005023807
- DE 10229732 A

**In der Beschreibung aufgeführte Nicht-Patentliteratur**

- **W. Mächtle.** *Makromolekulare Chemie,* 1984, vol. 185, 1025-1039
- **Houben-Weyl.** Methoden der organischen Chemie, Band XIV/1, Makromolekulare Stoffe. Georg-Thieme-Verlag, 1961, vol. XIV/1, 411-420